# FASCICULE DE BREVET EUROPEEN

(11) **EP 2 413 895 B1**
(45) Date de publication et mention de la délivrance du brevet: **26.12.2012**
(21) Numéro de dépôt: 10717674.5
(22) Date de dépôt: 30.03.2010
(51) Int. Cl.: A61K 47/32, A61K 8/81, A61Q 1/00, A61Q 5/00, A61Q 17/04, C08F 2/06, C08F 220/58, A61K 9/06, A61K 9/10, C08F 2/22, C08F 2/38, A61K 9/00, A61K 8/00, A61Q 19/00, A61K 9/107

(54) **NOUVEAU POLYMERE EPAISSISSANT SOUS FORME DE POUDRE**
NEUES VERDICKENDES POLYMER IN PULVERFORM
NOVEL THICKENING POLYMER IN THE FORM OF A POWDER

(30) Priorité: 31.03.2009 FR 0952019
(43) Date de publication de la demande: 08.02.2012
(73) Titulaire: Societe D'Exploitation De Produits Pour Les Industries Chimiques Seppic, 75007 Paris (FR)
(72) Inventeur: BRAUN, Olivier, F-81100 Castres (FR); MALLO, Paul, F-78290 Croissy- sur- Seine (FR)
(74) Mandataire: Conan, Philippe Claude
(86) Numéro de dépôt international: PCT/FR2010/050581
(87) Numéro de publication internationale: WO 2010/112753

(56) Documents cités:
- WO-A-00/38751
- FR-A- 2 910 899

## Description

L'invention concerne de nouveaux polymères, le procédé pour leur préparation et leur utilisation dans les formulations cosmétiques ou pharmaceutiques.

L'industrie cosmétique utilise très régulièrement des polymères épaississants synthétiques pour augmenter la viscosité des crèmes, émulsions et diverses solutions topiques.

Les polymères épaississants synthétiques actuellement utilisés dans cette industrie se présentent sous deux formes physiques, la forme poudre et la forme liquide dans laquelle le polymère est dispersé dans une émulsion eau dans huile à l'aide d'agents tensioactifs. Cette forme liquide est communément appelée latex inverse.

Les polymères épaississants sous forme de poudre utilisés dans cette industrie, sont principalement des polymères d'acide acrylique ou les copolymères d'acide acrylique et d'acrylate d'alkyle, comme ceux commercialisés sous les noms CARBOPOL™ et PEMULEN™ et décrits dans les brevets américains publiés sous les numéros US 5,373,044 et US 2,798,053, ou dans la demande de brevet européen publiée sous le numéro EP 301 532.

Plus récemment, on a aussi utilisé dans cette industrie des homopolymères de ou des copolymères de l'acide 2-acrylamido-2-méthyl propanesulfonique, eux-aussi sous forme de poudre. De tels produits sont commercialisés sous le nom ARISTOFLEX et décrits dans les demandes de brevet européen publiées sous les numéros EP 816 403, EP 1 116 733 et EP 1 069 142.

Tous les polymères décrits ci-dessus sont obtenus par polymérisation précipitante, à partir des monomères correspondants mis en solution dans un solvant organique comme le benzène, l'acétate d'éthyle, l'hexane, le cyclohexane, ou le tertio-butanol.

Ces épaississants sous forme de poudre permettent d'obtenir des gels clairs et ils sont généralement plutôt bien tolérés par la peau.

Par contre, ces épaississants sous forme de poudre présentent certains inconvénients, en ce qu'ils sont parfois difficiles à manipuler, qu'ils se dispersent ou se dissolvent lentement dans le milieu à épaissir, qu'ils comprennent souvent un résidu de solvant et que leur pouvoir épaississant est considérablement affecté par la présence des électrolytes dans la solution à épaissir.

L'industrie cosmétique utilise aussi très largement des épaississants sous forme de latex inverses, par exemple ceux commercialisés sous les noms SEPIGEL™ 305, SIMULGEL™ 600, SIMULGEL™ EG, SIMULGEL™ NS, SIMULGEL™ A, SEPIPLUS™ 400, ™ 265 et SIMULGEL™ S.

Contrairement aux polymères en poudre, les latex inverses sont faciles à utiliser et ils se dispersent très facilement en développant des performances épaississantes nettement supérieures.

Néanmoins, ces latex inverses présentent l'inconvénient de contenir une huile et un ou plusieurs agents tensioactifs nécessaires pour la bonne stabilité du produit vis à vis des phénomènes de décantation et les bonnes propriétés d'inversion requises lors de l'utilisation. Or, certaines de ces huiles et/ou tensioactifs induisent parfois des réactions d'intolérance cutanée. De plus ce type d'épaississants ne permet pas de préparer des gels clairs.

La demande de brevet européen publiée sous le numéro EP 1 496 081 divulgue des épaississants en poudre obtenus à partir de latex inverses après élimination d'huile et atomisation. Un épaississant de ce type est commercialisé sous le nom SEPINOV™ EMT 10. Bien que combinant une partie des avantages des épaississants en poudre classique, telle que l'absence d'huile, et les avantages des latex inverses, comme la grande vitesse de dissolution dans l'huile et un fort pouvoir épaississant, ils ne conduisent cependant pas à des gels parfaitement clairs et ne peuvent donc être utilisés dans la préparation des formulations cosmétiques transparentes. De plus, ils n'ont pas un comportement satisfaisant dans les compositions cosmétiques très riches en électrolytes, tels que les filtres solaires.

Ce dernier inconvénient, la résistance aux électrolytes, a pu être amélioré, en synthétisant des polymères en poudre de 2-acrylamido 2-méthyl propanesulfonate d'ammonium, de N,N-diméthyl acrylamide et de méthacrylate de béhényle pentacosaéthoxylé (25 OE), par polymérisation précipitante dans le tertio-butanol, tels que ceux décrits dans la demande internationale WO 2008/087326.

Cependant le tertio-butanol est un solvant cher et dont la purification, notamment l'élimination de l'eau formée lors du procédé de polymérisation, est complexe du fait de l'existence d'un azéotrope.

De plus, l'utilisation du tertio-butanol impose la neutralisation de l'acide 2 acrylamido 2 méthyl propanesulfonique par de l'ammoniac ou par un sel ammoniaqué, car ce sont les seuls sels de cet acide solubles dans le tertio-butanol. Or la présence d'ammonium reste un handicap en cosmétique, qu'il s'agisse de handicap de type "marketing" ou techniques comme le possible dégagement d'ammoniac de la formulation finale, les possibles réactions secondaires de l'ion ammonium avec certain principes actifs comme la dihydroxyacétone (DHA), de plus en plus utilisé dans les formulations auto-bronzantes.

La demande internationale 00/38751 divulgue un procédé de préparation de polymères sensibles aux ions qui sont insolubles dans des solutions aqueuses concentrées et solubles dans des solutions faiblement salées par polymerisation radicalaire de monomères sous forme acide libre, dans un mélange acétone eau (70/30 massique), suivie après formation du polymère, d'une éventuelle neutralisation du polymère obtenu.

C'est pourquoi les inventeurs ont cherché à synthétiser de nouveaux épaississants qui n'aient pas les inconvénients développés ci-dessus.

Selon un premier aspect, l'invention a pour objet un procédé de préparation d'un polyélectrolyte anionique linéaire branché ou réticulé, caractérisé en ce que ledit polyélectrolyte anionique comporte pour 100% molaire, jusqu'à 95% molaire d'unités monomériques issues de l'acide 2-méthyl 2-[(1-oxo 2-propènyl) amino] 1-propanesulfonique totalement salifié sous forme du sel sodium, jusqu'à 90% molaire d'au moins un monomère neutre, et optionnellement jusqu'à 10 % molaire d'au moins un monomère de formule (I) : 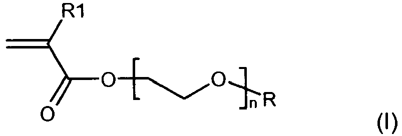 dans laquelle R1 représente un atome d'hydrogène ou un radical méthyle, R représente un radical alkyle linéaire ou ramifié comportant de huit à trente atomes de carbone et n représente un nombre supérieur ou égal à un et inférieur ou égal à cinquante, étant entendu que la somme des proportions molaires en unités monomériques constitutives dudit polyélectrolyte anionique indiquées ci-dessus n'excèdent pas 100% molaire, et en ce que ledit procédé comprend les étapes suivantes :

Une étape a) de préparation d'un mélange réactionnel comprenant dans les proportions souhaitées et dans un solvant (S), l'acide 2-méthyl 2-[(1-oxo 2-propènyl) amino] 1-propanesulfonique, le ou les monomères neutres, au moins un agent de neutralisation susceptible de former le sel de sodium dudit acide 2-méthyl 2-[(1-oxo 2-propènyl) amino] 1-propanesulfonique, le cas échéant le ou les monomères de formule (I) telle que définie précédemment, et, si nécessaire ou si désiré de l'agent réticulant et/ ou des autres additifs, ledit solvant (S) étant :
- soit une cétone de formule (II) : 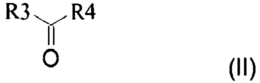 dans laquelle R3 et R4 identiques ou différents, représentent indépendamment l'un de l'autre un radical méthyle, un radical éthyle, un radical isopropyle ou un radical isobutyle ;
- soit un mélange consistant en, pour 100% molaire :
   - de l'eau en une proportion supérieure à 0% molaire et inférieure ou égale à 25% molaire ; et
   - une cétone de formule (II) telle que définie ci-dessus, en une proportion supérieure ou égal à 75% molaire et inférieure à 100% ;

Une étape b) au cours de laquelle la réaction de polymérisation est amorcée par introduction dans ledit mélange réactionnel préparé à l'étape a), d'un initiateur de radicaux libres, puis est laissée se dérouler jusqu'à sa conclusion, pour obtenir un précipité dudit polyélectrolyte anionique linéaire branché ou réticulé.

Le procédé tel que défini ci-dessus peut comprendre en outre une étape c) d'isolation dudit précipité obtenu à l'étape b) par séparation d'avec ledit solvant (S), puis si nécessaire ou si désiré, une étape d) de séchage dudit précipité résultant de l'étape c.

Par polyélectrolyte branché, on désigne un polyélectrolyte non linéaire qui possède des chaînes pendantes de manière à obtenir, lorsqu'il est mis en solution dans l'eau, un fort état d'enchevêtrement conduisant à des viscosités, à bas gradient de vitesse, très importantes.

Par polyélectrolyte réticulé, on désigne un polyélectrolyte non linéaire se présentant à l'état de réseau tridimensionnel insoluble dans l'eau, mais gonflable à l'eau et conduisant donc à l'obtention d'un gel chimique.

Le polyélectrolyte obtenu par le procédé selon l'invention peut comporter des motifs réticulés et/ou des motifs branchés.

Dans la formule (I) telle que définie précédemment, par radical alkyle linéaire ou ramifié comportant de huit à trente atomes de carbone, on désigne plus particulièrement pour R, ou bien une radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle, eicosyle ou docosyle ou triacontanyle ; ou bien un radical dérivé des alcools de Guerbet, qui sont des 1-alcanol ramifiés répondant à la formule générale :

CH₃-(CH2)ₚ-CH[CH₃-(CH₂)ₚ₋₂]-CH₂OH,

dans laquelle p représente un nombre entier compris entre 2 et 14, tels que, par exemple, les radicaux 2-éthyl hexyle, 2-propyl heptyle, 2-butyl octyle, 2-pentyl nonyle, 2-hexyl décyle ou 2-octyl dodécyle ; ou bien un radical dérivé des isoalcanols répondant à la formule générale :

CH₃-CH(CH₃)-(CH₂)ₘ-CH₂OH,

dans laquelle m représente un nombre entier compris entre 2 et 26 tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle.

Par au moins un agent de neutralisation susceptible de former le sel de sodium, on désigne dans le procédé tel que défini précédemment, notamment le carbonate de sodium, l'hydroxyde de sodium ou le bicarbonate de sodium (dénommé aussi hydrogénocarbonate de sodium).

Selon un aspect particulier du procédé tel que défini précédemment, l'agent de neutralisation est le bicarbonate de sodium.

Selon un autre aspect particulier de la présente invention, le procédé tel que défini précédemment est mis en oeuvre pour préparer un polyélectrolyte anionique, comportant des unités monomériques issues du composé de formule (I) dans laquelle R représente un radical alkyle comportant de 10 à 22 atomes de carbone, plus particulièrement un radical alkyle comportant de 12 à 22 atomes de carbone et tout particulièrement de 18 à 22 atomes de carbone.

Selon un autre aspect particulier de la présente invention, le procédé tel que défini précédemment est mis en oeuvre pour préparer un polyélectrolyte anionique, comportant des unités monomériques issues du composé de formule (I) dans laquelle n représente un nombre entier compris entre 3 et 30.

Selon un autre aspect particulier de la présente invention, le procédé tel que défini précédemment est mis en oeuvre pour préparer un polyélectrolyte anionique, comportant des unités monomériques issues du composé de formule (I) dans laquelle R1 représente un atome d'hydrogène.

Selon un autre aspect particulier de la présente invention, le procédé tel que défini précédemment est mis en oeuvre pour préparer un polyélectrolyte anionique, comportant des unités monomériques issues de l'acrylate de lauryle tétraéthoxylé, du méthacrylate de lauryle tétraéthoxylé, de l'acrylate de béhényle pentacosaéthoxylé ou du méthacrylate de béhényle pentacosaéthoxylé.

Selon un autre aspect particulier, l'invention a pour objet un procédé tel que défini ci-dessus, dans lequel ledit solvant (S) est :
- soit une cétone choisie parmi le propane-2-one, le butane-2-one, le pentane-2-one, le 3-méthyl butane-2-one, le 3-éthyl pentane-2-one ou le 4-méthyl pentane-2-one ;
- soit un mélange consistant en, pour 100% molaire :
   - de l'eau en une proportion supérieure à 0% molaire et inférieure ou égale à 10% molaire, de préférence inférieure ou égale à 5% molaire ; et
   - une cétone choisie parmi le propane-2-one, le butane-2-one, le pentane-2-one, le 3-méthyl butane-2-one, le 3-éthyl pentane-2-one ou le 4-méthyl pentane-2-one de formule (II) telle que définie précédemment, en une proportion molaire supérieure ou égale à 90% molaire, de préférence supérieure ou égale à 95% molaire et inférieure à 100% molaire.

Selon un aspect tout particulier, l'invention a pour objet le procédé tel que défini ci-dessus, dans lequel ledit solvant (S) est soit de l'acétone, soit un mélange eau-acétone en un ratio molaire eau/acétone supérieur à 0 et inférieur ou égal à 5/95.

Selon un autre aspect particulier de la présente invention, le procédé tel que défini précédemment est caractérisé en ce que ledit polyélectrolyte anionique ne comporte pas d'unités monomériques issues d'un composé de formule (I).

Selon un autre aspect particulier de la présente invention, le procédé tel que défini précédemment est caractérisé en ce que ledit polyélectrolyte anionique comporte pour 100% molaire, entre 0,05% molaire et 5% molaire, et plus particulièrement entre 0,1% molaire et 1% molaire, d'unités monomériques issues d'un composé de formule (I), telle que définie précédemment.

Le monomère neutre est notamment choisi parmi l'acrylamide, le méthacrylamide, les N-alkyl acrylamide, dans lesquels le groupe alkyle comporte de un à quatre atomes de carbone, comme par exemple le N-méthyl acrylamide, le N-éthyl acrylamide, le N-propyl acrylamide, le N-isopropyl acrylamide, le N-butyl acrylamide, le N-(tert-butyl) acrylamide, les N-alkyl méthacrylamide, dans lesquels le groupe alkyle comporte de un à quatre atomes de carbone, comme par exemple le N-méthyl méthacrylamide, le N-éthyl méthacrylamide, le N-propyl méthacrylamide, le N-isopropyl méthacrylamide, N-butyl méthacrylamide ou le N-(tert-butyl) méthacrylamide, les N,N-dialkyl acrylamide, dans lesquels chacun des groupes alkyle comportent entre un et quatre atomes de carbone, comme par exemple, le N,N-diméthyl acrylamide, le N,N-diéthyl acrylamide, le N,N-dipropyl acrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle), le diacétone acrylamide ou un dérivé éthoxylé de poids moléculaire compris entre 400 g/mol et 1000 g/mol, de chacun de ces esters, ou le vinyl pyrrolidone.

Selon un autre aspect particulier de la présente invention, le procédé tel que défini précédemment, est caractérisé en ce que ledit polyélectrolyte anionique comporte des unités monomériques issues d'un monomère neutre choisi parmi l'acrylamide, l'acrylate de (2-hydroxy éthyle) ou le N,N-diméthyl acrylamide.

Selon un aspect particulier de la présente invention, le procédé tel que défini précédemment est caractérisé en ce que ledit polyélectrolyte anionique comporte pour 100% molaire, plus de 10% molaire, de préférence plus de 25% molaire et jusqu'à 90% molaire d'unités monomériques issues de l'acide 2-méthyl 2-[(1-oxo 2-propènyl) amino] 1-propanesulfonique totalement salifié sous forme du sel sodium, de 5% molaire jusqu'à moins de 90% molaire, de préférence jusqu'à moins de 75% molaire, d'un monomère neutre et de 0% à 5% molaire d'un monomère de formule (I) telle que définie précédemment.

Selon un autre aspect particulier de la présente invention, le procédé tel que défini précédemment est caractérisé en ce que ledit polyélectrolyte anionique comporte pour 100% molaire, plus de 50% molaire, de préférence plus 75% molaire et jusqu'à 85% molaire d'unités monomériques issues de l'acide 2-méthyl 2-[(1-oxo 2-propènyl) amino] 1-propanesulfonique totalement salifié sous forme du sel sodium, de 10% molaire et jusqu'à moins de 50% molaire, de préférence moins de 25% molaire d'un monomère neutre et de 0% à 5% molaire d'un monomère de formule (I) telle que définie précédemment.

Selon un autre aspect particulier de la présente invention, le procédé tel que défini précédemment est caractérisé en ce que ledit polyélectrolyte anionique est réticulé et/ou branché avec un composé diéthylénique ou polyéthylènique dans la proportion molaire, exprimée par rapport l'ensemble des monomères mis en oeuvre, comprise entre 0,005% molaire et 1 % molaire, et de préférence de 0,01 % molaire à 0,5% molaire et plus particulièrement de 0,01% molaire à 0,25% molaire. Selon cet aspect particulier, l'agent de réticulation et/ou l'agent de ramification mis en oeuvre est choisi parmi le diméthacrylate d'éthylèneglycol, l'acide diallyloxacétique ou un de ses sels comme le diallyloxyacétate de sodium, le tétraallyloxyéthane, le diacrylate d'éthylèneglycol, le diallyl urée, le triallyl amine, le triacrylate de triméthylolpropane, le méthylène-bis(acrylamide) ou un mélange de ces composés.

Selon un autre aspect de la présente invention, le procédé tel que défini précédemment est mis en oeuvre pour préparer un terpolymère réticulé de l'acide 2-méthyl 2-[(1-oxo 2-propènyl) amino] 1-propanesulfonique salifié sous forme de sel de sodium, du N,N-diméthyl acrylamide et du méthacrylate de béhènyle pentacosaéthoxylé, dans lequel, pour 100% d'unités monomériques, plus de 75% et jusqu'à 85% molaire d'unités monomériques sont issues de l'acide 2-méthyl 2-[(1-oxo 2-propènyl) amino] 1-propanesulfonique totalement salifié sous forme du sel sodium, de 10% molaire jusqu'à moins de 25% molaire d'un monomère neutre et 0,05% molaire et 5% molaire d'un monomère de formule (I).

Selon un autre aspect, l'invention a aussi pour objet le terpolymère tel que défini ci-dessus.

Selon un autre aspect particulier de la présente invention, dans l'étape a) du procédé tel que défini précédemment, on ajoute un ou plusieurs additifs choisis parmi les agents complexant, les agents de transfert ou les agents limiteurs de chaîne.

Selon un autre aspect particulier de la présente invention, le procédé tel que défini précédemment est mis en oeuvre pour préparer les polyélectrolytes anioniques suivants :
- Copolymère d'acide 2-méthyl 2-[(1-oxo 2-propènyl) amino] 1-propanesulfonique partiellement salifié sous forme de sel de sodium et d'acrylamide réticulé au méthylène bis(acylamide) ;
- Copolymère d'acide 2-méthyl 2-[(1-oxo 2-propènyl) amino] 1-propanesulfonique partiellement salifié sous forme de sel de sodium et d'acrylamide réticulé au méthylène bis(acylamide)
- Copolymère d'acide 2-méthyl 2-[(1-oxo 2-propènyl) amino] 1-propanesulfonique partiellement salifié sous forme de sel de sodium et de N,N-diméthyl acrylamide réticulé au méthylène bis(acylamide)
- Terpolymère d'acide 2-méthyl 2-[(1-oxo 2-propènyl) amino] 1-propanesulfonique partiellement salifié sous forme de sel de sodium, d'acrylamide et d'acrylate de lauryle tétraéthoxylé, réticulé au méthylène-bis(acrylamide) ;
- Terpolymère d'acide 2-méthyl 2-[(1-oxo 2-propènyl) amino] 1-propanesulfonique partiellement salifié sous forme de sel de sodium, d'acrylate de 2-hydroxy éthyle et d'acrylate de lauryle tétraéthoxylé, réticulé au méthylène-bis(acrylamide) ;
- Terpolymère d'acide 2-méthyl 2-[(1-oxo 2-propènyl) amino] 1-propanesulfonique partiellement salifié sous forme de sel de sodium, d'acrylamide et de méthacrylate de béhènyle pentacosaéthoxylé, réticulé au méthylène-bis(acrylamide) ;
- Terpolymère d'acide 2-méthyl 2-[(1-oxo 2-propènyl) amino] 1-propanesulfonique partiellement salifié sous forme de sel de sodium, d'acrylate de 2-hydroxy éthyle et l'acrylate de béhènyle pentacosaéthoxylé, réticulé au triacrylate de triméthylolpropane ;
- Terpolymère d'acide 2-méthyl 2-[(1-oxo 2-propènyl) amino] 1-propanesulfonique partiellement salifié sous forme de sel de sodium, d'acrylamide et d'acrylate de béhènyle pentacosaéthoxylé, réticulé au méthylène-bis(acrylamide) ;
- Terpolymère d'acide 2-méthyl 2-[(1-oxo 2-propènyl) amino] 1-propanesulfonique partiellement salifié sous forme de sel de sodium, de N,N-diméthyl acrylamide et d'acrylate de lauryle tétraéthoxylé, réticulé au méthylène-bis(acrylamide) ;
- Terpolymère d'acide 2-méthyl 2-[(1-oxo 2-propènyl) amino] 1-propanesulfonique partiellement salifié sous forme de sel de sodium, de N,N-diméthyl acrylamide et de méthacrylate de lauryle tétraéthoxylé, réticulé au méthylène-bis(acrylamide) ;
- Terpolymère d'acide 2-méthyl 2-[(1-oxo 2-propènyl) amino] 1-propanesulfonique partiellement salifié sous forme de sel de sodium, de N,N-diméthyl acrylamide et de méthacrylate de béhènyle pentacosaéthoxylé, réticulé au méthylène-bis(acrylamide) ;
- Terpolymère d'acide 2-méthyl 2-[(1-oxo 2-propènyl) amino] 1-propanesulfonique partiellement salifié sous forme de sel de sodium, de N,N-diméthyl acrylamide et de méthacrylate de béhènyle pentacosaéthoxylé, réticulé au triacrylate de triméthylolpropane.

Selon un autre aspect particulier de la présente invention, dans l'étape b) du procédé tel que défini précédemment, la réaction de polymérisation est amorcée à une température égale ou supérieure à 50°C à l'aide d'un amorceur radicalaire produisant des radicaux par homolyse, tel que le peroxyde de dilauroyle, l'azo-bis(isobutyronitrile) ou encore les dérivés azoïques.

Selon un autre aspect particulier de la présente invention, dans l'étape b) du procédé tel que défini précédemment, la réaction de polymérisation est amorcée par un couple oxydo-réducteur, à une température inférieure ou égale à 20°C, puis conduite de manière quasi-adiabatique.

Selon un autre aspect particulier de la présente invention, dans l'étape c) du procédé tel que défini précédemment, la séparation du précipité obtenu dudit solvant organique est effectuée par filtration.

L'invention a aussi pour objet une variante du procédé tel que défini précédemment, caractérisée en ce qu'elle comprend, à la place de l'étape a), une étape a2) de préparation d'un mélange réactionnel comprenant dans les proportions souhaitées et dans un solvant (S), le ou les monomères neutres, le sel de sodium dudit acide 2-méthyl 2-[(1-oxo 2-propènyl) amino] 1-propanesulfonique, le cas échéant le ou les monomères de formule (I) telle que définie précédemment et plus particulièrement une variante du procédé tel que défini ci-dessus, caractérisée en ce qu'elle comprend, préalablement à l'étape a2), une étape a1) de préparation du sel de sodium de l'acide 2-méthyl 2-[(1-oxo 2-propènyl) amino] 1-propanesulfonique, par neutralisation dudit acide 2-méthyl 2-[(1-oxo 2-propènyl) amino] 1-propanesulfonique avec un agent de neutralisation susceptible de former le sel de sodium dudit acide 2-méthyl 2-[(1-oxo 2-propènyl) amino] 1-propanesulfonique.

Selon un autre aspect, l'invention a pour objet l'utilisation du polyélectrolyte anionique obtenu par le procédé tel que défini précédemment ou terpolymère tel que défini ci-dessus, comme épaississant et/ou comme stabilisant et/ou émulsionnant, d'une composition topique cosmétique, dermopharmaceutique ou pharmaceutique.

Selon un autre aspect, l'invention a pour objet une composition topique cosmétique, dermopharmaceutique ou pharmaceutique comprenant une quantité efficace de polyélectrolyte anionique obtenu par le procédé tel que défini précédemment ou du terpolymère tel que défini précédemment.

Par quantité efficace, on entend une quantité suffisante pour obtenir un épaississement satisfaisant de ladite composition topique cosmétique, dermopharmaceutique ou pharmaceutique.

Selon un autre aspect particulier de la présente invention, ladite composition topique cosmétique, dermopharmaceutique ou pharmaceutique comprend entre 0,1 % et 10 % massique et plus particulièrement de 0,5% à 5% massique du polyélectrolyte anionique obtenu par le procédé tel que défini précédemment ou du terpolymère tel que défini précédemment.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile-dans-eau (H/E), eau-dans-huile (E/H), huile-dans-eau-dans-huile (H/E/H) ou eau-dans-huile-dans-eau (E/H/E). La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple consister en un agent anti-inflammatoire, un myorelaxant, un antifongique, un antibactérien ou antipelliculaire.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent autobronzant, un filtre solaire, un antirides, un agent à visée amincissante, un agent antiradicalaire, un agent anti-acnéique, un antifongique ou antipelliculaire.

Selon un dernier aspect particulier, l'invention a pour objet une composition topique cosmétique, telle que définie précédemment comprenant en outre de 0,5 % à 5% massique de dihydroxyacétone.

Le pH de la composition topique est de préférence supérieur ou égal à 3.

La composition topique peut en outre comporter des composés classiquement compris dans ce type de compositions, par exemple des parfums, des conservateurs, des colorants, des pigments, des écrans solaires, des ingrédients actifs, des émollients ou des tensioactifs.

Selon un autre aspect particulier, l'invention concerne l'utilisation du polyélectrolyte anionique tel que défini précédemment, pour épaissir, émulsionner et stabiliser une composition topique comprenant au moins une phase aqueuse.

Le polyélectrolyte anionique selon l'invention, est un substitut intéressant aux latex inverses vendus sous les noms de SEPIGEL™ 305, SEPIGEL™ 501, SIMULGEL™ EG, SIMULGEL™ EPG, SIMULGEL™ NS, SIMULGEL™ 600, SIMULGEL™ A, SEPIPLUS™ 265, SEPIPLUS™ 250, SEPIPLUS™ 400 ou SEPINOV™ EMT 10 par la demanderesse, car il présente aussi une bonne compatibilité avec les autres excipients utilisés pour la préparation de formulations telles que les laits, les lotions, les crèmes, les savons, les bains, les baumes, les shampooings ou les après-shampooings. II peut aussi être mis en oeuvre avec lesdits SEPIGEL™ ou SIMULGEL™, SEPIPLUS™ et/ou SEPINOV™ EMT 10.

Il est notamment compatible avec les concentrés décrits et revendiqués dans les publications internationales WO 92/06778, WO 95/04592, WO 95/13863, WO 96/37285, WO 98/22207, WO 98/47610 ou dans FR 2 734 496, avec les agents tensioactifs décrits dans WO 93/08204.

Il est particulièrement compatible avec le MONTANOV™ 68, le MONTANOV™ 82, le MONTANOV™ 202, le MONTANOV™ L, le MONTANOV™ S, le FLUIDANOV™ 20X ou l'EASYNOV™. Il peut également être utilisé dans des émulsions du type de celles décrites et revendiquées dans EP 0 629 396 et dans les dispersions aqueuses cosmétiquèrent ou physiologiquement acceptable avec un composé organopolysiloxane choisi, par exemple parmi ceux décrits dans WO 93/05762 ou dans WO 93/21316.

Il peut également être utilisé pour former des gels aqueux à pH acide cosmétiquement ou physiologiquement acceptables, tels que ceux décrit dans WO 93/07856; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019, ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 19523596.

Le polyélectrolyte selon l'invention est également compatible avec les principes actifs tels que par exemple, les agents auto-bronzants comme le dihydroxyacétone (DHA), l'érythrulose ou les agents anti-acné ; il peut donc être introduit dans des compositions auto-bronzantes comme celles revendiquées dans EP 0 715 845, EP 0 604 249, EP 0 576 188 ou dans WO 93/07902.

Il est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou WO 98/09611.

Il est également compatible avec des polymères épaississants et/ou gélifiants comme les hydrocolloïdes d'origine végétale ou biosynthétique, par exemple la gomme de xanthane, la gomme de karaya, les carraghénates, les alginates, les galactomannanes ; comme les silicates ; comme la cellulose et ses dérivés ; comme l'amidon et ses dérivés hydrophiles ; comme les polyuréthanes.

Les exemples suivants illustrent l'invention sans toutefois la limiter.

### A) Terpolymère ATBS (sel de Na)/DMAM/(BEM 25) réticulé au triacrvlate de triméthylolpropane selon le procédé obiet de l'invention (polyélectrolyte 1)

1) - On charge dans un réacteur verre de 2 litres à 20°C :
   - 344 g d'acétone,
   - 50,8 g d'acide 2-acrylamido 2-méthyl propanesulfonique (ATBS G)
   - 20,8 g de NaHCO₃
   - 6,1 g de N,N-diméthyl acrylamide
   - 9,6 g d'eau permutée
   - 0,58 g de méthacrylate de béhényle pentacosaéthoxylé (BEM-25)
   - 0,45 g de triacrylate de triméthylolpropane.
2) - Le réacteur est mis sous agitation puis un fort barbotage d'azote est appliqué pendant 1 h. Le milieu réactionnel est ensuite chauffé à 55°C puis 0,75 g de peroxyde de dilauroyle sont ajoutés pour amorcer la polymérisation.
   Le milieu épaissit rapidement puis redevient plus fluide après 30 min.
3) - Après deux heures de maintien au reflux de l'acétone, le milieu réactionnel est refroidi.
4) - Après filtration, la poudre de polyélectrolyte (1) recueillie est séchée dans l'étude sous vide.

### Evaluation des propriétés du polymère obtenu

Viscosité à 25°C de la solution de poudre à 2% dans l'eau (Brookfield RVT, Mobile 6, vitesse 5) : η = 60 200 mPa.s

Viscosité à 25°C de la solution de poudre à 2% dans l'eau + 0,1% NaCl (Brookfield RVT, Mobile 6, vitesse 5) : η = 71 000 mPa.s

Viscosité à 25°C de la solution de poudre à 2% dans l'eau + 1% NaCl (Brookfield RVT, Mobile 6, vitesse 5) : η = 60 200 mPa.s

Une tentative consistant à reproduire l'étape 1) du paragraphe A) précédent, pour préparer une solution comprenant du 2-acrylamido 2-méthyl propanesulfonate de sodium à 15,5% massique dans un mélange tert-butanol - eau (97,5/2,5 Vol/Vol) s'est soldée par un échec, le sel de sodium de l'acide 2-acrylamido 2-méthyl propanesulfonate tendant à former un précipité et/ou une dispersion instable dans le tert-butanol, rendant impossible une polymérisation homogène avec les autres monomères solubles de ce solvant.

### B) Exemples de formulations préparées avec le polyélectrolyte 1 préparé par le procédé selon l'invention

### Exemple 1 : Crème de soin

| | | |
|---|---|---|
| Cyclométhicone : | | 10% |
| Polyélectrolyte 1 : | | 0,8% |
| MONTANOV™ 68 : | | 2% |
| Alcool stéarylique : | | 1% |
| Alcool stéarique : | | 0,5% |
| Conservateur : | | 0,65% |
| Lysine : | | 0,025% |
| EDTA (sel disodique) : | | 0,05% |
| Gomme de xanthane : | | 0,2% |
| Glycérine : | | 3% |
| Eau : | q.s.p. | 100% |

### Exemple 2 : Baume après-rasage

### FORMULE

| | | | |
|---|---|---|---|
| A | Polyélectrolyte 1 : | | 1,5% |
| | Eau : | q.s.p. | 100% |
| B | MICROPEARL™ M 100 : | | 5,0% |
| | SEPICIDE™ CI : | | 0,50% |
| | Parfum : | | 0,20% |
| | Ethanol 95° : | | 10,0% |

### MODE OPERATOIRE : Ajouter B dans A.

### Exemple 3 : Emulsion satinée pour le corps

### FORMULE

| | | |
|---|---|---|
| A | SIMULSOL™ 165: | 5,0% |
| | LANOL™ 1688 : | 8,50% |
| | Beurre de Karité : | 2% |
| | Huile de paraffine : | 6,5% |
| | LANOL™ 14M : | 3% |
| | LANOL™ S : | 0,6% |
| B | Eau : | 66,2% |
| C | MICROPEARL™ M 100 : | 5% |
| D | Polyélectrolyte 1 : | 3% |
| E | SEPICIDE™ CI : | 0,3% |
| | SEPICIDE™ HB : | 0,5% |
| | AQUAXYL™ : | 3% |
| | Parfum : | 0,20% |
| | Acétate de vitamine E : | 0,20% |
| | Pyrolidinonecarboxylate de sodium : | 1% |

MODE OPERATOIRE : Ajouter C dans B, émulsionner B dans A à 70°C, puis ajouter D à 60°C puis E à 30°C.

### Exemple 4 : Crème H/E

### FORMULE

| | | | |
|---|---|---|---|
| A | SIMULSOL™ 165 : | | 5,0% |
| | LANOL™ 1688 : | | 20,0% |
| | LANOL™ P : | | 1,0% |
| B | Eau : | q.s.p. | 100% |
| C | Polyélectrolyte 1 : | | 2,50% |
| D | SEPICIDE™ CI : | | 0,20% |
| | SEPICIDE™ HB : | | 0,30% |

MODE OPERATOIRE : Introduire B dans A vers 75°C ; ajouter C vers 60°C, puis D vers 45°C.

### Exemple 5 : Gel solaire non gras

### FORMULE

| | | | |
|---|---|---|---|
| A | Polyélectrolyte 1 : | | 3,00% |
| | Eau : | | 30% |
| B | SEPICIDE™ CI : | | 0,20% |
| | SEPICIDE™ HB : | | 0,30% |
| | Parfum : | | 0,10% |
| C | Colorant : | | q.s. |
| | Eau: | | 30% |
| D | MICROPEARL™ M 100 : | | 3,00% |
| | Eau : | q.s.p | 100% |
| E | Huile de silicone : | | 2,0% |
| | PARSOL™ MCX : | | 5,00% |

MODE OPERATOIRE
Introduire B dans A ; ajouter C, puis D, puis E.

### Exemple 6 : Lait solaire

### FORMULE

| | | | |
|---|---|---|---|
| A | MONTANOV™ S : | | 3,0% |
| | Huile de sésame : | | 5,0% |
| | PARASOL™ MCX : | | 5,0% |
| | Carraghénane λ : | | 0,10% |
| B | Eau : | q.s.p. | 100% |
| C | Polyélectrolyte 1 : | | 0,80% |
| D | Parfum : | | q.s. |
| | Conservateur : | | q.s. |

MODE OPERATOIRE : Emulsionner B dans A à 75°C puis ajouter C vers 60°C, puis D vers 30°C et ajuster le pH si nécessaire.

### Exemple 7 : Gel de massage

### FORMULE

| | | |
|---|---|---|
| A | Polyélectrolyte 1 : | 3,5% |
| | Eau : | 20,0% |
| B | Colorant : | 2 gouttes/100g |
| | Eau : | q.s. |
| C | Ethanol: | 10% |
| | Menthol : | 0,10% |
| D | Huile de silicone : | 5,0% |

MODE OPERATOIRE : Ajouter B dans A, puis ajouter au mélange, C puis D.

### Exemple 8 : Fond de teint hydratant et matifiant

### FORMULE

| | | | |
|---|---|---|---|
| A | eau : | | 20,0% |
| | Butylène glycol : | | 4,0% |
| | PEG-400: | | 4,0% |
| | PECOSIL™ PS100 : | | 1,0% |
| | Hydroxyde de Sodium : | | q.s. pH = 9 |
| | Dioxyde de titane : | | 7,0% |
| | Talc : | | 2,0% |
| | Oxyde de fer jaune : | | 0,8% |
| | Oxyde de fer rouge : | | 0,3% |
| | Oxyde de fer noir : | | 0,05% |
| B | LANOL™ 99 : | | 8% |
| | Caprylic capric triglycéride | | 8% |
| | MONTANOV™ 202 : | | 5,00% |
| C | eau : | q.s.p. | 100% |
| | MICROPEARL™ M305 : | | 2,0% |
| | EDTA tétrasodé : | | 0,05% |
| D | Cyclométhicone : | | 4,0% |
| | Gomme de Xanthane : | | 0,2% |
| | Polyélectrolyte 1 : | | 0,8% |
| E | SEPICIDE™ HB : | | 0,5% |
| | SEPICIDE CI : | | 03% |
| | Parfum : | | 0,2% |

MODE OPERATOIRE : Préparer à 80°C, les mélanges B + D et A + C, puis mélanger et émulsionner l'ensemble.

### Exemple 9 : Gel coup d'éclat

| | | | |
|---|---|---|---|
| A | Polyélectrolyte 1 : | | 4% |
| | Eau : | | 30% |
| B | ELASTINE HPM : | | 5,0% |
| C | MICROPEARL™ M 100 : | | 3% |
| | Eau : | | 5% |
| D | SEPICIDE™ CI : | | 0,2% |
| | SEPICIDE™ HB : | | 0,3% |
| | Parfum : | | 0,06% |
| | Pyrolidinonecarboxylate de sodium 50% : | | 1% |
| | Eau : | q. s. p. | 100% |

MODE OPERATOIRE : Préparer A ; additionner B, puis C, puis D.

### Exemple 10 : Lait corporel

| | | |
|---|---|---|
| MONTANOV™ S : | | 3,5% |
| LANOL™ 37T : | | 8,0% |
| SOLAGUM™ L : | | 0,05% |
| Eau: | q.s.p. | 100% |
| Benzophénone-3 : | | 2,0% |
| Diméthicone 350cPs : | | 0,05% |
| Polyélectrolyte 1 : | | 0,8% |
| Conservateur : | | 0,2% |
| Parfum : | | 0,4% |

### Exemple 11 : Emulsion démaquillante à l'huile d'amandes douces

| | | |
|---|---|---|
| MONTANOV™ 68 : | | 5% |
| Huile d'amandes douces : | | 5% |
| Eau : | q.s.p. | 100% |
| Polyélectrolyte 1 : | | 0,3% |
| Glycérine : | | 5% |
| Conservateur : | | 0,2% |
| Parfum : | | 0,3% |

### Exemple 12 : Crème hydratante pour peaux grasses

| | | |
|---|---|---|
| MONTANOV™ 68 : | | 5% |
| Cétylstéaryloctanoate : | | 8% |
| Palmitate d'octyle : | | 2% |
| Eau : | q.s.p. | 100% |
| Polyélectrolyte 1 : | | 0,6% |
| MICROPEARL™ M100 : | | 3,0% |
| Mucopolysaccharides : | | 5% |
| SEPICIDE™ HB : | | 0,8% |
| Parfum : | | 0,3% |

### Exemple 13 : Baume après-rasage apaisant sans alcool

| | | |
|---|---|---|
| LIPACIDE™ PVB : | | 1,0% |
| LANOL™ 99 : | | 2,0% |
| Huile d'amandes douces : | | 0,5% |
| Polyélectrolyte 1 : | | 3,5% |
| Eau : | q.s.p. | 100% |
| Parfum: | | 0,4% |
| SEPICIDE™ HB : | | 0,4% |
| SEPICIDE™ CI : | | 0,2% |

### Exemple 14 : Crème aux AHA pour peaux sensibles

| | | |
|---|---|---|
| Mélange de N-lauroyl aminoacides : | | 0,1% à 5% |
| Aspartate de magnésium et de potassium : | | 0,002% à 0,5% |
| LANOL™ 99: | | 2% |
| MONTANOV™ 68 : | | 5,0% |
| Eau: | q.s.p. | 100% |
| Polyélectrolyte 1 : | | 1,50% |
| Acide gluconique : | | 1,50% |
| Triéthanolamine (TEA) : | | 0,9% |
| SEPICIDE™ HB : | | 0,3% |
| SEPICIDE™ Cl : | | 0,2% |
| Parfum : | | 0,4% |

### Exemple 15 : Soin apaisant après soleil

| | | |
|---|---|---|
| Mélange de N-lauryl aminoacides : | | 0,1% à 5% |
| Aspartate de magnésium et de potassium : | | 0,002% à 0,5% |
| LANOL™ 99 : | | 10,0% |
| Eau : | q.s.p. | 100% |
| Polyélectrolyte 1 : | | 2,50% |
| SEPICIDE™ HB : | | 0,3% |
| SEPICIDE™ CI : | | 0,2% |
| Parfum : | | 0,4% |
| Colorant : | | 0,03% |

### Exemple 16 : Lait démaquillant

| | | |
|---|---|---|
| MONTANOV™ S : | | 3% |
| PRIMOL™ 352 : | | 8,0% |
| Huile d'amandes douces : | | 2% |
| Eau : | q.s.p. | 100% |
| Polyélectrolyte 1 : | | 0,8% |
| Conservateur : | | 0,2% |

### Exemple 17 : Emulsion fluide à pH alcalin

| | | |
|---|---|---|
| MARCOL™ 82 : | | 5,0% |
| Hydroxyde sodium : | | 10,0% |
| Eau : | q.s.p. | 100% |
| Polyélectrolyte 1 : | | 1,5% |

### Exemple 18 : Fond de teint fluide

| | | |
|---|---|---|
| SIMULSOL™ 165 : | | 5,0% |
| LANOL™ 84D : | | 8,0% |
| LANOL™ 99 : | | 5,0% |
| Eau : | q.s.p. | 100% |
| Pigments et charges minérales : | | 10,0% |
| Polyélectrolyte 1 : | | 1,2% |
| Conservateur : | | 0,2% |
| Parfum: | | 0,4% |

### Exemple 19 : Lait solaire

| | | |
|---|---|---|
| MONTANOV™ S : | | 3,5% |
| LANOL™ 37T : | | 10,0% |
| PARSOL™ MCX : | | 5,0% |
| EUSOLEX™ 4360 : | | 2,0% |
| Eau : | q.s.p. | 100% |
| Polyélectrolyte 1 : | | 1,8% |
| Conservateur : | | 0,2% |
| Parfum : | | 0,4% |

### Exemple 20 : Gel contour des yeux

| | | |
|---|---|---|
| Polyélectrolyte 1 : | | 2,0% |
| Parfum : | | 0,06% |
| Pyrrolidinonecarboxylate de sodium : | | 0,2% |
| DOW CORNING™ 245 Fluid : | | 2,0% |
| Eau : | q. s. p. | 100% |

### Exemple 21 : Composition de soin non rincée

| | | |
|---|---|---|
| Polyélectrolyte 1 : | | 1,5% |
| Parfum : | | q. s |
| Conservateur : | | q. s. |
| DOW CORNING™ X2 8360 : | | 5,0% |
| DOW CORNING™ Q2 1401 : | | 15,0% |
| Eau : | q.s.p. | 100% |

### Exemple 22 : Gel amincissant

| | | |
|---|---|---|
| Polyélectrolyte 1 : | | 5% |
| Ethanol : | | 30% |
| Menthol : | | 0,1% |
| Caféine : | | 2,5% |
| Extrait de ruscus : | | 2% |
| Extrait de lierre : | | 2% |
| SEPICIDE™ HB : | | 1% |
| Eau: | q.s.p. | 100% |

### Exemple 23: Gel crème teinté ultra naturel

### FORMULE

| | | | |
|---|---|---|---|
| A | Eau : | | 10,0% |
| | Butylène glycol : | | 4,0% |
| | PEG-400: | | 4,0% |
| | PECOSIL™ PS100 : | | 1,5% |
| | NaOH : | q.s. | pH = 7 |
| | Dioxyde de titane : | | 2,0% |
| | Oxyde de fer jaune : | | 0,8% |
| | Oxyde de fer rouge : | | 0,3% |
| | Oxyde de fer noir : | | 0,05% |
| B | LANOL™ 99 : | | 4,0% |
| | Caprylic capric triglycéride | | 4,0% |
| | SEPIFEEL™ ONE : | | 1,0% |
| | Polyélectrolyte 1 : | | 3,0% |
| C | Eau: | q.s.p. | 100% |
| | MICROPEARL™ M305 : | | 2,0% |
| | EDTA tétrasodé : | | 0,05% |
| | Cyclométhicone : | | 4,0% |
| D | SEPICIDE™ HB : | | 0,5% |
| | SEPICIDE CI : | | 0,3% |
| | Parfum : | | 0,2% |

MODE OPERATOIRE : Préparer le mélange B + C puis ajouter A puis D.

### Exemple 24 : Soin pour les peaux grasses

| | | |
|---|---|---|
| MICROPEARL™ M310 : | | 1,0% |
| Polyélectrolyte 1 : | | 5,0% |
| Isononanoate d'octyle : | | 4.0% |
| Eau: | q.s.p. | 100% |
| SEPICONTROL™ A5 : | | 4,0% |
| Parfum: | | 0,1% |
| SEPICIDE™ HB : | | 0,3% |
| SEPICIDE™ Cl : | | 0,2% |
| CAPIGEL™ 98 : | | 0,5% |
| Eau : | | 10% |

### Exemple 25 : Crème aux AHA

| | | |
|---|---|---|
| MONTANOV™ 68 : | | 5,0% |
| LIPACIDE™ PVB: | | 1,05% |
| LANOL™ 99 : | | 10,0% |
| Eau : | q.s.p. | 100% |
| Acide gluconique : | | 1,5% |
| TEA (triéthanolamine) : | | 0,9% |
| Polyélectrolyte 1 : | | 1,5% |
| Parfum: | | 0,4% |
| SEPICIDE™ HB: | | 0,2% |
| SEPICIDE™ Cl: | | 0,4% |

### Exemple 26 : Autobronzant non gras pour visage et corps

| | | |
|---|---|---|
| LANOL™ 2681 : | | 3,0% |
| Polyélectrolyte 1 : | | 2,5% |
| Eau : | q.s.p. | 100% |
| Dihydroxyacétone : | | 3,0% |
| Parfum : | | 0,2% |
| SEPICIDE™ HB : | | 0,8% |
| Hydroxyde de sodium) : | q.s. | pH = 5 |

### Exemple 27 : Lait solaire au monoï de Tahiti

| | | |
|---|---|---|
| Monoï de Tahiti: | | 10% |
| LIPACIDE™ PVB : | | 0,5% |
| Polyélectrolyte 1 : | | 2,2% |
| Eau : | q.s.p. | 100% |
| Parfum : | | 0,1% |
| SEPICIDE™ HB : | | 0,3% |
| SEPICIDE™ CI : | | 0,1% |
| PARSOL™ MCX : | | 4,0% |

### Exemple 28 : Soin solaire pour le visage

| | | |
|---|---|---|
| Cyclométhicone et Diméthiconol : | | 4,0% |
| Polyélectrolyte 1 : | | 3,5% |
| Eau : | q.s.p. | 100% |
| Parfum : | | 0,1% |
| SEPICIDE™ HB : | | 0,3% |
| SEPICIDE™ Cl : | | 0,21% |
| PARSOL™ MCX : | | 5,0% |
| Micatitane : | | 2,0% |
| Acide lactique : | | q.s.p. pH = 6,5 |

### Exemple 29 : Emulsion bronzante sans soleil

| | | |
|---|---|---|
| LANOL™ 99 : | | 15% |
| MONTANOV™ 68 : | | 5,0% |
| PARSOL™ MCX : | | 3,0% |
| Eau: | q.s.p. | 100% |
| Dihydroxyacétone : | | 5,0% |
| Phosphate monosodique : | | 0,2% |
| Polyélectrolyte 1 : | | 0,5% |
| Parfum: | | 0,3% |
| SEPICIDE™ HB : | | 0,8% |
| Hydroxyde de sodium : | q.s. | pH=5. |

### Exemple 30 : Crème de soin

| | | |
|---|---|---|
| Cyclométhicone : | | 10% |
| Polyélectrolyte 1 : | | 0,8% |
| MONTANOV™ 68 : | | 4,5% |
| Conservateur : | | 0,65% |
| Lysine : | | 0,025% |
| EDTA (sel disodique) : | | 0,05% |
| Gomme de xanthane : | | 0,2% |
| Glycérine : | | 3% |
| Eau : | qsp | 100% |

### Exemple 31 : Crème de soin

| | | |
|---|---|---|
| Cyclométhicone : | | 10% |
| Polyélectrolyte 2 : | | 0,8% |
| MONTANOV™ 68 : | | 4,5% |
| Perfluoropolyméthylisopropyléther : | | 0,5% |
| Conservateur : | | 0,65% |
| Lysine : | | 0,025% |
| EDTA (sel disodique) : | | 0,05% |
| PEMULEN™ TR1 : | | 0,2% |
| Glycérine : | | 3% |
| Eau : | qsp | 100% |

### Exemple 32 : Lait corporel

### FORMULE

| | | | |
|---|---|---|---|
| A | SIMULSOL™ 165 : | | 5,0% |
| | LANOL™ 1688 : | | 12,0% |
| | LANOL™ 14 M : | | 2,0% |
| | Alcool cétylique : | | 0,3% |
| | SCHERCEMOL™ OP : | | 3% |
| B | Eau: | q.s.p. | 100% |
| C | Polyélectrolyte 1 : | | 0,35% |
| D | SEPICIDE™ CI : | | 0,2% |
| | SEPICIDE™ HB : | | 0,5% |
| | Parfum : | | 0,20% |

MODE OPERATOIRE : Emulsionner B dans A vers 75°C ; ajouter C vers 60°C, puis D vers 30°C.

### Exemple 33 : Gel soin de massage

### FORMULE

| | | | |
|---|---|---|---|
| A | Polyélectrolyte 1 : | | 3,00% |
| | Eau : | | 30% |
| B | SEPICIDE™ CI : | | 0,20% |
| | SEPICIDE™ HB : | | 0,30% |
| | Parfum: | | 0,05% |
| C | Colorant : | | q.s. |
| | Eau : | q.s.p. | 100% |
| D | MICROPEARL™ SQL : | | 5,0% |
| | LANOL™ 1688 : | | 2% |

MODE OPERATOIRE : Préparer A ; additionner B, puis C, puis D.

### Exemple 34 : Lait corporel

### FORMULE

| | | | |
|---|---|---|---|
| A | MONTANOV™ S : | | 3,0% |
| | Triheptonate de glycérol : | | 10,0% |
| | B Eau : | q.s.p. | 100% |
| C | Polyélectrolyte 1 : | | 1,0% |
| D | Parfum : | | q.s. |
| | Conservateur : | | q.s. |

MODE OPERATOIRE : Fondre A à environ 75°C. Emulsionner B dans A à 75°C puis ajouter C vers 60°C, puis D.

### Exemple 35 : Baume après-rasage apaisant sans alcool

| | | |
|---|---|---|
| Mélange de lauryl aminoacides : | | 0,1% à 5% |
| Aspartate de magnésium et de potassium : | | 0,002% à 0,5% |
| LANOL™ 99 : | | 2% |
| Huile d'amandes douces : | | 0,5% |
| Eau : | q.s.p. | 100% |
| Polyélectrolyte 1 : | | 3% |
| SEPICIDE™ HB : | | 0,3% |
| SEPICIDE™ CI : | | 0,2% |
| Parfum : | | 0,4% |

### Exemple 36: Lait corporel

| | | |
|---|---|---|
| MONTANOV™ S : | | 3,5% |
| LANOL™ 37T : | | 8,0% |
| SOLAGUM™ L : | | 0,05% |
| Eau : | q.s.p. | 100% |
| Benzophénone-1 : | | 2,0% |
| Diméthicone 350 cPs : | | 0,05% |
| Polyélectrolyte 2 : | | 0,8% |
| Conservateur : | | 0,2% |
| Parfum : | | 0,4% |

### Exemple 37 : Baume après-rasage apaisant sans alcool

| | | |
|---|---|---|
| LIPACIDE™ PVB : | | 1,0% |
| LANOL™ 99 : | | 2,0% |
| Huile d'amandes douces : | | 0,5% |
| Polyélectrolyte 1 : | | 3,5% |
| Eau : | q.s.p. | 100% |
| Parfum : | | 0,4% |
| SEPICIDE™ HB : | | 0,4% |
| SEPICIDE™ CI : | | 0,2% |

### Exemple 38 : Gel rafraîchissant après-rasage

| | | |
|---|---|---|
| LIPACIDE™ PVB : | | 0,5% |
| LANOL™ 99 : | | 5,0% |
| Polyélectrolyte 1 : | | 2,5% |
| Eau : | q.s.p. | 100% |
| MICROPEARL™ LM : | | 0,5% |
| Parfum : | | 0,2% |
| SEPICIDE™ HB : | | 0,3% |
| SEPICIDE™ CI : | | 0,2% |

### Exemple 39 : Crème aux AHA

| | | |
|---|---|---|
| MONTANOV™ 68 : | | 5,0% |
| LIPACIDE™ PVB : | | 1,05% |
| LANOL™ 99 : | | 10,0% |
| Eau: | q.s.p. | 100% |
| Acide gluconique : | | 1,5% |
| TEA (triéthanolamine) : | | 0,9% |
| Polyélectrolyte 1 : | | 1,5% |
| Parfum: | | 0,4% |
| SEPICIDE™ HB : | | 0,2% |
| SEPICIDE™ CI : | | 0,4% |

### Exemple 40 : Gel brillance

| | | |
|---|---|---|
| Polyélectrolyte 1 : | | 1,5% |
| Silicone volatile: | | 25% |
| Monopropylèneglycol : | | 25% |
| Eau déminéralisée : | | 10% |
| Glycérine : | qsp | 100% |

### Exemple 41 : Gel amincissant

| | | |
|---|---|---|
| Polyélectrolyte 1 : | | 1,5% |
| Isononanoate d'isononyle : | | 2% |
| Caféine : | | 5% |
| Ethanol : | | 40% |
| MICROPEARL™ LM : | | 2% |
| Eau déminéralisée : | qsp | 100% |
| Conservateur parfum : | | qs |

### Exemple 42 : Lait démaquillant

| | | |
|---|---|---|
| SIMULSOL™ 165: | | 4% |
| MONTANOV™ 202 : | | 1% |
| Caprylate-caprate triglyceride : | | 15% |
| PECOSIL™ DCT : | | 1% |
| Eau déminéralisée : | | qs |
| CAPIGEL™ 98 : | | 0,5% |
| Polyélectrolyte 1 : | | 1% |
| PROTEOL™ APL : | | 2% |
| Hydroxyde de sodium : | qsp | pH = 7 |

### Exemple 43 : Masque crème "rince off" restructurant pour cheveux stressés et fragilisés

| | | |
|---|---|---|
| KETROL™T : | | 0,5% |
| PECOSIL™ SPP50 : | | 0,75% |
| N-cocoyl aminoacides : | | 0,70% |
| Butylèneglycol : | | 3,0% |
| Polyélectrolyte 1 : | | 3,0% |
| MONTANOV™ 82 : | | 3,0% |
| Huile de jojoba : | | 1,0% |
| LANOL™ P : | | 6,0% |
| AMONYL™ DM : | | 1,0% |
| LANOL™ 99 : | | 5,0% |
| SEPICIDE™ HB : | | 0,3% |
| SEPICIDE™Cl : | | 0,2% |
| Parfum: | | 0,2% |
| Eau : | qsp | 100% |

### Exemple 44 : Crème solaire

| | | |
|---|---|---|
| SIMULSOL™ 165 : | | 3% |
| MONTANOV™ 202 : | | 2% |
| Benzoate C12-C15 : | | 8% |
| PECOSIL™ PS 100 : | | 2% |
| Diméthicone : | | 2% |
| Cyclométhicone : | | 5% |
| Para-méthoxy cinnamate d'octyle : | | 6% |
| Benzophénone-3 : | | 4% |
| Oxyde de Titane : | | 8% |
| Gomme xanthane : | | 0,2% |
| Butylèneglycol : | | 5% |
| Eau déminéralisée : | qsp | 100% |
| Polyélectrolyte 1 : | | 1,5% |
| Conservateur, parfum : | | qs |

### Exemple 45 : Gel de soin peaux mixtes

| | | |
|---|---|---|
| Polyélectrolyte 1 : | | 4% |
| Squalane végétal : | | 5% |
| Diméthicone : | | 1,5% |
| SEPICONTROL™ A5 : | | 4% |
| Gomme xanthane : | | 0,3% |
| Eau : | qsp | 100% |
| Conservateur, Parfum : | | qs. |

### Exemple 46 : Lotion capillaire

| | | |
|---|---|---|
| Butylène glycol : | | 3,0% |
| Polyélectrolyte 1 : | | 3% |
| SIMULSOL™ 1293 : | | 3,0% |
| Acide lactique : | qs | pH = 6 |
| SEPICIDE™ HB : | | 0,2% |
| SEPICIDE™ Cl : | | 0,3% |
| Parfum : | | 0,3% |
| Eau : | qs | 100% |

### Exemple 47 : Shampooing protecteur et relaxant

| | | |
|---|---|---|
| Amonyl™ 675 SB : | | 5,0% |
| Sodium lauryl éther sulfate à 28% : | | 35,0% |
| Polyélectrolyte 1 : | | 3,0% |
| SEPICIDE™ HB: | | 0,5% |
| SEPICIDE™Cl : | | 0,3% |
| Hydroxyde de sodium : | q.s. | pH = 7,2 |
| Parfum: | | 0,3% |
| Colorant (FDC bleu 1/jaune 5) : | | q.s. |
| Eau : | QSP | 100% |

### Exemple 48 : Protecteur "leave-on"; Soin antistress pour cheveux

| | |
|---|---|
| KETROL™T : | 0,5% |
| Mélange de cocoyl aminoacides : | 3,0% |
| Butylèneglycol : | 5,0% |
| DC 1501 : | 5,0% |
| Polyélectrolyte 1 : | 4,0% |
| SEPICIDE™ HB: | 0,5% |
| SEPICIDE™Cl : | 0,3% |
| Parfum : | 0,3% |
| Eau : | QSP 100 |

### Exemple 49 : Crème vitaminée

| | | |
|---|---|---|
| SIMULSOL™ 165 : | | 5% |
| MONTANOV™ 202 : | | 1% |
| Caprylic/capric triglycérides : | | 20% |
| Palmitate de vitamine A : | | 0,2% |
| Acétate de vitamine E : | | 1 % |
| MICROPEARL™ M 305 : | | 1,5% |
| Polyélectrolyte 1 : | | 2% |
| Eau | qsp | 100% |
| Conservateur, parfum | | qs |

### Exemple 50 : Gel solaire

| | | |
|---|---|---|
| Polyélectrolyte 1 : | | 3,00% |
| SEPICIDE™ CI : | | 0,20% |
| SEPICIDE™ HB : | | 0,30% |
| Parfum : | | 0,10% |
| Colorant : | | qs |
| Silice : | | 3,00% |
| Eau: | q.s.p | 100% |
| Huile de silicone : | | 2,0% |
| Benzophénone-3 : | | 5,00% |

### Exemple 51 : Gloss lèvre

| | | |
|---|---|---|
| Polyélectrolyte 1 : | | 1,50% |
| Schercemol™ TISC : | | 15,00% |
| Vistanol™ NPGC : | | 15,00% |
| Candurin Paprika : | | 0,50% |
| MONTANOX™ 80 : | | 1,00% |
| Antaron™ V216 : | | 0,90% |
| Arôme Abricot : | | 0,20% |
| SEPICIDE™ HB : | | 0,50% |
| C Maltidex™ H16322: | qsp | 100% |

### Exemple 52 : Poudre pressée Terre de soleil

| | | |
|---|---|---|
| Polyélectrolyte 1 : | | 2,00% |
| LANOLT™ 99 : | | 12,00% |
| SEPIWHITE™ MSH : | | 1,00% |
| Talc : | | 33,00% |
| MICROPEARL™ M310 : | | 3,00% |
| Oxyde de fer jaune : | | 0,80% |
| Oxyde de fer rouge : | | 0,30% |
| Oxyde de fer noir : | | 0,05% |
| Mica : | qs | 100% |

### Exemple 53 : Emulsion pour peaux à tendance atopique

| | | |
|---|---|---|
| ARLACELT™ P135 : | | 2,00% |
| Polyélectrolyte 1 : | | 1,00% |
| LANOL™ 1688 : | | 14,00% |
| PRIMOL™ 352 : | | 8,00% |
| Glycérine : | | 5,00% |
| Eau : | qsp | 100% |
| Sulfate de magnésium : | | 0,70% |
| SEPICIDE™ HB : | | 0,30% |
| SEPICIDE™ Cl : | | 0,20% |
| MICROPEARL™ M310 : | | 5,00% |

### Exemple 54 : Soin solaire apaisant (eau dans silicone)

| | | |
|---|---|---|
| Polyélectrolyte 1 : | | 2,00% |
| DC5225C : | | 20,00% |
| DC345 : | | 10,00% |
| SEPICALM™ VG : | | 3,00% |
| Dioxyde de titane MT100T : | | 5,00% |
| Oxyde de zinc Z cote HP1 : | | 5,00% |
| SEPICIDE™ HB : | | 0,30% |
| Parfum : | | 0,05% |
| SEPICIDE™ Cl : | | 0,20% |
| Glycérine : | | 5,00% |
| Chlorure de sodium : | | 2,00% |
| Eau : | qsp | 100% |

### Exemple 55 : Soin multi-phases

| | |
|---|---|
| Polyélectrolyte 1 : | 3,00% |
| C12-15 alkylbenzoate : | 25,00% |
| AQUAXYL™ : | 3,00% |
| SEPITONIC™ M3: | 1,00% |
| SEPICIDE™ HB : | 0,50% |
| SEPICIDE™ Cl : | 0,30% |

### Exemple 56 : Gel autobronzant

| | | |
|---|---|---|
| Polyélectrolyte 1 : | | 5,0% |
| Ethanol : | | 30% |
| Dihydroxyacétone : | | 5% |
| Menthol : | | 0,1% |
| Caféine : | | 2,5% |
| Extrait d'Ivry : | | 2% |
| SEPICIDE™ HB : | | 1% |
| Eau : | qsp | 100% |

### Exemple 57 : Gel solaire et autobronzant

| | | |
|---|---|---|
| MONTANOV™ S : | | 3,0% |
| Triheptanoate de glycéryle : | | 10,0% |
| LIPACIDE™ PVB : | | 1,05% |
| Polyélectrolyte 1 : | | 2,2% |
| Eau : | qs | 100% |
| Dihydroxyacétone : | | 5% |
| Parfum : | | 0,1% |
| SEPICIDE™ HB : | | 0,3% |
| SEPICIDE™ Cl : | | 0,1% |
| PARSOL™ MCX : | | 4,0% |

### Exemple 58 : Crème auto-bronzante aux α-hydroxy acides

| | | |
|---|---|---|
| MONTANOV™ 68 : | | 5,0% |
| LIPACIDE™ PVB : | | 1,05% |
| LANOL™ 99 : | | 10,0% |
| Eau : | qs | 100% |
| Acide gluconique : | | 1,5% |
| Dihydroxyacétone : | | 3% |
| Triéthanolamine : | | 0,9% |
| Polyélectrolyte 1 : | | 1,5% |
| Parfum : | | 0,4% |
| SEPICIDE™ HB : | | 0,2% |
| SEPICIDE™ Cl : | | 0,4% |

### Exemple 59 : Crème auto-bronzante aux α-hydroxy acides pour peau sensible

| | | |
|---|---|---|
| Mélange de N-lauroyl aminoacides : | | 0,1% à 5% |
| Aspartate de magnésium et de potassium : | | 0,002% à 0,5% |
| MONTANOV™ 68 : | | 5,0% |
| LANOL™ 99 : | | 2,0% |
| Eau : | qs | 100% |
| Acide lactique : | | 1,5% |
| Dihydroxyacétone : | | 3,5% |
| Triéthanolamine : | | 0,9% |
| Polyélectrolyte 1 : | | 1,5% |
| Parfum : | | 0,4% |
| SEPICIDE™ HB : | | 0,3% |
| SEPICIDE™ CI : | | 0,2% |

### Exemple 60 : Emulsion hydratante auto-bronzante satinée

| | |
|---|---|
| SIMULSOL™ 165 : | 5,0% |
| LANOL™ 1688 : | 8,5% |
| Beurre de Galam : | 2% |
| Paraffine liquide : | 6,5% |
| LANOL™ 14M : | 3% |
| LANOL™ S : | 0,6% |
| Eau : | 66,2% |
| Dihydroxyacétone : | 3% |
| MICROPEARL™ M 100 : | 5% |
| Polyélectrolyte 1 : | 3% |
| 0AQUAXYL™ : | 5% |
| Acétate de vitamine E : | 0,20% |
| Pyrolidinonecarboxylate de sodium : | 0,20% |
| Parfum : | 0,2% |
| SEPICIDE™ HB : | 0,5% |
| SEPICIDE™ CI : | 0,3% |

Les définitions des produits commerciaux utilisés dans les exemples, sont les suivantes :
SIMULSOL™ 1293 est de l'huile de castor hydrogénée et éthoxylée, avec un indice d'éthoxylation égal à 40, commercialisé par la société SEPPIC.
CAPIGEL™ 98 est un épaississant liquide à base de copolymère acrylate commercialisé pas la société SEPPIC.
KETROL™ T est de la gomme de xanthane commercialisée par la société KELCO.
LANOL™ 99 est de l'isononanoate d'isononyle commercialisé par la société SEPPIC.
DC1501 est un mélange de cyclopentasiloxane et de diméthiconol commercialisé par la société DOW CHEMICAL.
MONTANOV™ 82 est un agent émulsionnant à base d'alcool cétéarylique et de cocoylglucoside.
Le MONTANOV^{™} 68 (cétéaryl glucoside) est une composition auto-émulsionnable telle que décrite dans WO 92/06778, commercialisée par la société SEPPIC.
Le MICROPEARL^{™} M 100 est une poudre ultra fine au toucher très doux et à action matifiante commercialisée par la société MATSUMO.
Le SEPICIDE™ Cl, imidazolidine urée, est un agent conservateur commercialisé par la société SEPPIC.
PEMULEN™ TR1 est un polymère acrylique commercialisé par GOODRICH.
Le SIMULSOL™ 165 est du stéarate de glycérol auto-émulsionnable commercialisé par la société SEPPIC.
Le LANOL™ 1688 est un ester émollient à effet non gras commercialisé par la société SEPPIC.
Le LANOL™ 14M et le LANOL™ S sont des facteurs de consistance commercialisés par la société SEPPIC.
Le SEPICIDE™ HB, qui est un mélange de phénoxyéthanol, de méthylparaben, d'éthylparaben, de propylparaben et de butylparaben, est un agent conservateur commercialisé par la société SEPPIC.
L'AQUAXYL™ est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
Le PARSOL™ MCX est du para-méthoxy cinnamate d'octyle commercialisé par la société GIVAUDAN.
Le MONTANOV™ S est un agent nacrant, commercialisé par la société SEPPIC, à base d'un mélange d'alkyl poly glucosides tels que ceux décrits dans WO 95/13863.
Le MICROPEARL™ SQL est un mélange de micro particules renfermant du squalane qui se libère sous l'action du massage ; il est commercialisé par la société MATSUMO.
Le LANOL™ 37T est du triheptanoate de glycérol, commercialisé par la société SEPPIC.
Le SOLAGUM™ L est un carraghénane commercialisé par la société SEPPIC.
Le MARCOL™ 82 est une huile de paraffine commercialisée par la société EXXON.
Le LANOL™ 84D est du malate de dioctyle commercialisé par la société SEPPIC.
Le PARASOL™ NOX est un filtre solaire commercialisé par la société GIVAUDAN.
l'EUSOLEX™ 4360 est un filtre solaire commercialisé par la société MERCK.
Le DOW CORNING™ 245 Fluid est de la cyclométhicone, commercialisée par la société DOW CORNING.
Le LIPACIDE™ PVB, est un hydrolysât de protéines de blé acylé commercialisé par la société SEPPIC.
Le MICROPEARL™ LM est un mélange de squalane, de polyméthylméthacrylate et de menthol, commercialisé par la société SEPPIC.
Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.
Le LANOL™ 2681 est un mélange caprylate, caprate de coprah, commercialisé par la société SEPPIC.
Le MONTANOV™ 202, est une composition APG/alcools gras telle que décrite dans WO9 98/47610, commercialisée par la société SEPPIC.
Le PROTEOL™ APL est un tensioactif moussant, commercialisé par la société SEPPIC.
Le SCHERCEMOL™ TISC est un ester (citrate de tri-isostéaryle) commercialisé par la société SCHER.
Le VISTANOL™ NPGC est un ester (néopentyl glycol dicaprate) commercialisé par la société SEWA KASEI.
L'ANTARON™ V216 est un polymère synthétique (PVP/hexadecene copolymer) distribué par la société UNIVAR.
Le C MALTIDEX™ H16322 est polyol (sirop de maltitol) commercialisé par la société CERESTAR.
Le SEPIWHITE™MSH est un actif dépigmentant (N-undecylenoyl phenylalanine) commercialisé par la société SEPPIC.
Le DC 345 est un cyclométhicone commercialisé par la société Dow Corning.
Le DC 5225C est un mélange de cyclopentasiloxane et de diméthicone copolyol commercialisé par la société DOW CORNING.
Le SEPICALM™ VG est un actif apaisant (sodium palmitoylproline) commercialisé par la société SEPPIC.
Le MT100VT est un dioxyde de titane micronisé ayant subi un traitement de surface (hydroxyde d'aluminium / acide stéarique) distribué par la société UNIPEX.
Le Z COTE HP1 est un oxyde de zinc micronisé ayant subi un traitement de surface distribué par GATTEFOSSE.
Le CANDURIN PAPRIKA est un mélange de silicate de potassium et d'aluminium et d'oxyde de fer.
Le MICROPEARL™ M 310 est une poudre ultra fine au toucher très doux et à action matifiante commercialisée par la société MATSUMO.
Le PRIMOL™ 352 est une huile minérale commercialisée par la société EXXON.
Le PECOSIL™DCT est du sodium Diméthicone PEG-7 Acetyl Methyltaurate commercialisé par la société PHOENIX.
Le PECOSIL™PS 100 est du Diméthicone PEG-7 commercialisé par la société PHOENIX.

## Revendications

1. Procédé de préparation d'un polyélectrolyte anionique linéaire branché ou réticulé, **caractérisé en ce que** ledit polyélectrolyte anionique comporte pour 100% molaire, jusqu'à 95% molaire d'unités monomériques issues l'acide 2-méthyl 2-[(1-oxo 2-propènyl) amine] 1-propanesulfonique totalement salifié sous forme du sel sodium, jusqu'à 90% molaire d'au moins un monomère neutre, et optionnellement jusqu'à 10 % molaire d'au moins un monomère de formule (I) : 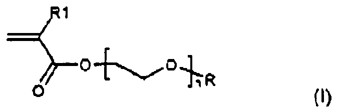 dans laquelle R1 représente un atome d'hydrogène ou un radical méthyle, R représente un radical alkyle linéaire ou ramifié comportant de huit à trente atomes de carbone et n représente un nombre supérieur ou égal à un et inférieur ou égal à cinquante, étant entendu que la somme des proportions molaires en unités monomériques constitutives dudit polyélectrolyte anionique indiquées ci-dessus n'excédent pas 100% molaire, et **en ce que** ledit procédé comprend les étapes suivantes :
Une étape a) de préparation d'un mélange réactionnel comprenant dans les proportions souhaitées et dans un solvant (S), l'acide 2-méthyl 2-[(1-oxo 2-propènyl) amino] 1-propanesulfonique, le ou les monomères neutres, au moins un agent de neutralisation susceptible de former le sel de sodium dudit acide 2-méthyl 2-[(1-oxo 2-propènyl) amino] 1-propanesulfonique, le cas échéant le ou les monomères de formule (I) telle que définie précédemment, et, si nécessaire ou si désiré, de l'agent réticulant et/ ou des autres additifs, ledit solvant (S) étant :
- soit une cétone de formule (II) : 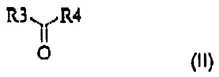 dans laquelle R3 et R4 identiques ou différents, représentent indépendamment l'un de l'autre un radical méthyle, un radical éthyle, un radical isopropyle ou un radical isobutyle;
- soit un mélange consistant en, pour 100% molaire :
- de l'eau en une proportion supérieure à 0% molaire et inférieure ou égale à 25% molaire, et
- une cétone de formule (II) telle que définie ci-dessus, en une proportion supérieure ou égal à 75% molaire et inférieure à 100% ;
Une étape b) au cours de laquelle la réaction de polymérisation est amorcée par Introduction dans ledit mélange réactionnel préparé à l'étape a), d'un initiateur de radicaux libres, puis est laissée se dérouler jusqu'à sa conclusion, pour obtenir un précipité dudit polyélectrolyte anionique linéaire branché ou réticulé.

2. Procédé tel que défini à la revendication 1 comprenant en outre une étape c) d'isolation dudit précipité obtenu à l'étape b) par séparation d'avec ledit solvant (S).

3. Procédé tel que défini à la revendication 2 comprenant en outre une étape d) de séchage dudit précipité résultant de l'étape c).

4. Procédé tel que défini à l'une des revendications 1 à 3, dans lequel ledit solvant (S) est :
- soit une cétone choisie parmi le propane-2-one, le butane-2-one, le pentane-2-one, le 3-méthyl butane-2-0ne, le 3-éthyl pentane-20-one ou la 4-méthyl pentane-2-one ;
- soit un mélange consistant en, pour 100% molaire :
- de l'eau en une proportion supérieure à 0% molaire et inférieure ou égale à 15% molaire, de préférence Inférieure ou égale à 5% molaire ; et
- une cétone choisie parmi le propane-2-one, le butane-2-one, le pentane-2-one, le 3-méthyl butane-2-one, le 3-éthyl pentane-2-one ou le 4-méthyl pentane-2-one de formule (II) telle que définie précédemment, en une proportion molaire supérieure ou égale à 90% molaire, de préférence supérieure ou égale à 95% molaire et inférieure à 100% molaire.

5. Procédé tel que défini à la revendication 4, dans lequel ledit solvant (S) est soit de l'acétone, soit un mélange eau-acétone en un ratio molaire eau/acétone supérieur à 0 et inférieur ou égal à 5/95.

6. Procédé tel que défini à l'une des revendications 1 à 5, **caractérisé en ce que** ledit polyélectrolyte anionique, comporte pour 100% molaire, entre 0,05% molaire et 5% molaire, et plus particulièrement entre 0,1% molaire et 1% molaire, d'unités monomériques issues d'un composé de formule (I),

7. Procédé tel que défini à l'une des revendications 1 à 6, **caractérisé en ce que** ledit polyélectrolyte anionique, comporte des unités monomériques issues d'un monomère neutre choisi parmi l'acrylamlde, l'acrylate de (2-hydroxy éthyle) ou le N,N-dlméthyl acrylamide.

8. Procédé tel que défini à l'une des revendications 1 à 7, **caractérisé en ce que** ledit polyélectrolyte anionique comporte pour 100% molaire, plus de 10% molaire, de préférence plus de 25% molaire et jusqu'à 90°% molaire d'unités monomériques issues de l'acide 2-méthyl 2-[(1-oxo 2-propènyl) amino] 1-propanssulfonique totalement salifié sous forme du sel sodium, de 5% molaire jusqu'à moins de 90% molaire, de préférence jusqu'à moins de 75% molaire, d'un monomère neutre et de 0% à 6% molaire d'un monomère de formule (I),

9. Procédé tel que défini à la revendication 8, **caractérisé en ce que** ledit polyélectrolyte anionique comporte pour 100% molaire, plus de 50% molaire, de préférence plus de 75% molaire et jusqu'à 85% molaire d'unités monomériques issues de l'acide 2-méthyl 2-[(1-oxo 2-propènyl) amino] 1-propanesulfanique totalement salifié sous forme du sel sodium, de 10% molaire et jusqu'à moins de 50% molaire, de préférence moins de 25% molaire d'un monomère neutre et de 0% à 5% molaire d'un monomère de formule (I).

10. Procédé tel que défini à l'une des revendications 1 à 9, **caractérisé en ce que** ledit polyélectrolyte anionique est réticulé et/ou branché avec un composé diéthylénique ou polyéthylènique dans la proportion molaire, exprimée par rapport à l'ensemble des monomères mis en oeuvre, comprise entre 0,005% molaire et 1% molaire.

11. Variante du procédé tel que défini à l'une des revendications 1 à 10, **caractérisée en ce qu'**elle comprend, à la place de l'étape a), une étape a2) de préparation d'un mélange réactionnel comprenant dans les proportions souhaitées et dans un solvant (S), le ou les monomères neutres, le sel de sodium dudit acide 2-méthyl 2-[(1-oxo 2-propènyl) amino] 1-propanesulfonique, le cas échéant le ou les monomères de formule (I) telle définie précédemment.

12. Variante du procédé telle que définie à la revendication 11, **caractérisée en ce qu'**elle comprend, préalablement à l'étape a2), une étape a1) de préparation du sel de sodium de l'acide 2-méthyl 2-[(1-oxo 2-propènyl) amino] 1-propanesulionique, par neutralisation dudit acide 2-méthyl 2-[(1-oxo 2-propènyl) amino] 1-propanesulfonique avec un agent de neutralisation susceptible de former le sel de sodium dudit acide 2-méthyl 2-[(1-oxo 2-propènyl) amino] 1-propanesulfonique.

13. Procédé ou sa variante tel que défini à l'une des revendications 1 à 12, **caractérisé en ce que** ledit polyélectrolyte anionique réticulé est un terpolymère réticulé de acide 2-méthyl 2-[(1-oxo 2-propènyl) amine] 1-propanesulfonique salifié sous forme de sel de sodium, du N,N-diméthyl acrylamide et du méthacrylate de béhènyle pentacosaéthoxylé, dans lequel pour 100% d'unités monomériques, plus de 75% et jusqu'à 85% molaire d'unités monomériques sont issues de l'acide 2-méthyl 2-[(1-oxo 2-propènyl)amino]1-propanesulfonique totalement salifié sous forme du sel sodium, de 10% molaire Jusqu'à moins de 25% molaire d'un monomère neutre et 0,05% molaire et 5% molaire d'un monomère de formule (I).

## Claims

1. Process for preparing a linear, branched or crosslinked anionic polyelectrolyte, **characterised in that** said anionic polyelectrolyte comprises, for 100 mol %, up to 95 mol % monomeric units derived from 2-methyl-2-[(1-oxo-2-propenyl)amino]-1-propanesulphonic acid which is totally salified in the form of the sodium salt, up to 90 mol % of at least one neutral monomer, and optionally up to 10 mol % of at least one monomer of formula (I): 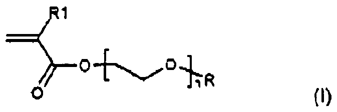 wherein R1 is a hydrogen atom or a methyl radical, R is a linear or branched alkyl radical comprising from eight to thirty carbon atoms, and n is a number greater than or equal to one and less than or equal to fifty, it being understood that the sum of the molar proportions of constituent monomeric units of said anionic polyelectrolyte that are indicated above does not exceed 100 mol %, and **in that** said process comprises the following steps:
a step a) of preparing a reaction mixture comprising, in the desired proportions and in a solvent (S), 2-methyl-2-[(1-oxo-2-propenyl)amino]-1-propanesulphonic acid, the neutral monomer(s), at least one neutralising agent capable of forming the sodium salt of said 2-methyl-2-[(1-oxo-2-propenyl)amino]-1-propanesulphonic acid, optionally the monomer(s) of formula (I) as defined above, and, if necessary or if desired, crosslinking agent and/or other additives, said solvent (S) being:
- either a ketone of formula (II): 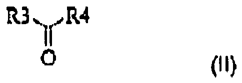 wherein R3 and R4, which may be identical or different, are, independently of one another, a methyl radical, an ethyl radical, an isopropyl radical or an isobutyl radical;
- or a mixture consisting of, for 100 mol %:
- water in a proportion greater than 0 mol % and less than or equal to 25 mol %; and
- a ketone of formula (II) as defined above, in a proportion greater than or equal to 75 mol % and less than 100 %;
a step b) during which the polymerisation reaction is initiated by introducing, into said reaction mixture prepared in step a), a free-radical initiator and is then left to continue to the conclusion thereof, so as to obtain a precipitate of said linear, branched or crosslinked anionic polyelectrolyte.

2. Process according to claim 1, also comprising a step c) of isolating said precipitate obtained in step b) by separation from with said solvent (S).

3. Process according to claim 2, also comprising a step d) of drying said precipitate resulting from step c).

4. Process according to any of claims 1 to 3, wherein said solvent (S) is:
- either a ketone selected from propan-2-one, butan-2-one, pentan-2-one, 3-methylbutan-2-one, 3-ethylpentan-20-one or 4-methylpentan-2-one;
- or a mixture consisting of, for 100 mol %:
- water in a proportion of greater than 0 mol % and less than or equal to 15 mol %, preferably less than or equal to 5 mol %; and
- a ketone selected from propan-2-one, butan-2-one, pentan-2-one, 3-methylbutan-2-one, 3-ethylpentan-2-one or 4-methylpentan-2-one, of formula (II) as defined above, in a molar proportion of greater than or equal to 90 mol %, preferably greater than or equal to 95 mol % and less than 100 mol %.

5. Process according to claim 4, wherein said solvent (S) is either acetone, or a water/acetone mixture in a water/acetone molar ratio of greater than 0 and less than or equal to 5/95.

6. Process according to any of claims 1 to 5, **characterised in that** said anionic polyelectrolyte comprises, for 100 mol %, between 0.05 mol % and 5 mol %, and more particularly between 0.1 mol % and 1 mol %, monomeric units derived from a compound of formula (I).

7. Process according to any of claims 1 to 6, **characterised in that** said anionic polyelectrolyte comprises monomeric units derived from a neutral monomer selected from acrylamide, (2-hydroxyethyl) acrylate or N,N-dimethylacrylamide.

8. Process according to any of claims 1 to 7, **characterised in that** said anionic polyelectrolyte comprises, for 100 mol %, more than 10 mol %, preferably more than 25 mol % and up to 90 mol % monomeric units derived from 2-methyl-2-[(1-oxo-2-propenyl)amino]-l-propanesulphonic acid which is totally salified in the form of the sodium salt, from 5 mol % up to less than 90 mol %, preferably up to less than 75 mol %, of a neutral monomer, and from 0 mol % to 5 mol % of a monomer of formula (I).

9. Process according to claim 8, **characterised in that** said anionic polyelectrolyte comprises, for 100 mol %, more than 50 mol %, preferably more than 75 mol %, and up to 85 mol % monomeric units derived from 2-methyl-2-[(1-oxo-2-propenyl)amino]-1-propanesulphonic acid which is totally salified in the form of the sodium salt, from 10 mol % and up to less than 50 mol %, preferably less than 25 mol %, of a neutral monomer, and from 0 mol % to 5 mol % of a monomer of formula (I).

10. Process according to any of claims 1 to 9, **characterised in that** said anionic polyelectrolyte is crosslinked and/or branched with a diethylene or polyethylene compound in the molar proportion, expressed relative to all the monomers used, of between 0.005 mol % and 1 mol %.

11. Variant of the process according to any of claims 1 to 10, **characterised in that** it comprises, in place of step a), a step a2) of preparing a reaction mixture comprising, in the desired proportions and in a solvent (S), the neutral monomer(s), the sodium salt of said 2-methyl-2-[(1-oxo-2-propenyl)amino]-1-propanesulphonic acid, and optionally the monomer(s) of formula (I) as defined above.

12. Variant of the process according to claim 11, **characterised in that** it comprises, prior to step a2), a step a1 of preparing the sodium salt of 2-methyl-2-[(l-oxo-2-propenyl)amino]-l-propanesulphonic acid by neutralising said 2-methyl-2-[(1-oxo-2-propenyl)amino]-1-propanesulphonic acid with a neutralising agent capable of forming the sodium salt of said 2-methyl-2-[(1-oxo-2-propenyl)amino]-1-propanesulphonic acid.

13. Process or the variant thereof according to any of claims 1 to 12, **characterised in that** said crosslinked anionic polyelectrolyte is a crosslinked terpolymer of 2-methyl-2-[(1-oxo-2-propenyl)amino]-1-propanesulphonic acid which is salified in the form of the sodium salt, of N,N-dimethylacrylamide and of pentacosaethoxylated behenyl methacrylate, wherein, for 100 % monomeric units, more than 75 mol % and up to 85 mol % monomeric units are derived from 2-methyl-2-[(1-oxo-2-propenyl)amino]-1-propanesulphonic acid which is totally salified in the form of the sodium salt, from 10 mol % up to less than 25 mol % from a neutral monomer, and 0.05 mol % and 5 mol % from a monomer of formula (I).

## Patentansprüche

1. Verfahren zur Herstellung eines linearen, verzweigten oder vernetzten anionischen Polyelektrolyten, **dadurch gekennzeichnet, dass** der anionische Polyelektrolyt auf 100 Mol-% bis zu 95 Mol-% monomere Einheiten, die von 2-Methyl-2-[(1-oxo-2-propenyl)amino]-1-propansulfonsäure stammen, die in Form des Natriumsalzes vollständig zu Salz umgewandelt ist, bis zu 90 Mol-% von mindestens einem neutralen Monomer und optional bis zu 10 Mol-% von mindestens einem Monomer mit der Formel (I) aufweist: 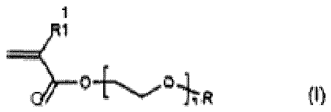 wobei R1 ein Wasserstoffatom oder einen Methylrest darstellt, R einen linearen oder verzweigten Alkylrest darstellt, der von acht bis dreißig Kohlenstoffatome aufweist, und n eine Zahl größer als oder gleich eins und kleiner als oder gleich fünfzig darstellt, wobei die Summe der zuvor angegebenen molaren Anteile der zuvor angegebenen monomeren Einheiten, die den anionischen Polyelektrolyten bilden, 100 Mol-% nicht zu übersteigen hat, und dadurch, dass das Verfahren folgende Schritte umfasst:
einen Schritt a) zum Herstellen eines Reaktionsgemischs, das in den gewünschten Verhältnissen und in einem Lösungsmittel (S) 2-Methyl-2-[(1-oxo-2-propenyl)amino]-1-propansulfonsäure, das oder die neutrale(n) Monomer(e), mindestens ein Neutralisationsmittel, das in der Lage ist, das Natriumsalz der 2-Methyl-2-[(1-oxo-2-propenyl)amino]-1-propansulfonsäure zu bilden, gegebenenfalls das oder die Monomere mit der Formel (I), wie zuvor definiert, und, wenn erforderlich oder wenn gewünscht, Vernetzungsmittel und/oder weitere Zusatzstoffe umfasst, wobei das Lösungsmittel (S):
- entweder ein Keton mit der Formel (II): 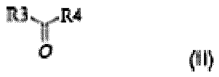 ist, wobei R3 und R4, identisch oder unterschiedlich, unabhängig voneinander einen Methylrest, einen Ethylrest, einen Isopropylrest oder einen Isobutylrest darstellen;
- oder ein Gemisch ist, das auf 100 Mol-% aus Folgendem besteht:
- Wasser in einem Anteil von über 0 Mol-% und unter oder gleich 25 Mol-%, und
- einem Keton mit der Formel (II), wie zuvor definiert, in einem Anteil von über oder gleich 75 Mol-% und unter 100 %;
einen Schritt b), während dessen die Polymerisationsreaktion durch Einbringen eines Radikalinitiators in das Reaktionsgemisch, das in Schritt a) hergestellt wurde, gestartet wird, anschließend bis zum Ende laufen gelassen wird, um einen Niederschlag des linearen verzweigten oder vernetzten anionischen Polyelektrolyten zu erhalten.

2. Verfahren nach Anspruch 1, das ferner einen Schritt c) zum Isolieren des Niederschlags, der in Schritt b) erhalten wurde, durch Trennung von mit dem Lösungsmittel (S) umfasst.

3. Verfahren nach Anspruch 2, das ferner einen Schritt d) zum Trocknen des Niederschlags, der aus Schritt c) hervorgeht, umfasst.

4. Verfahren nach einem der Ansprüche 1 bis 3, wobei das Lösungsmittel (S):
- entweder ein Keton ist, das aus Propan-2-on, Butan-2-on, Pentan-2-on, 3-Methyl-butan-2-on, 3-Ethyl-pentan-20-on oder 4-Methyl-pentan-2-on ausgewählt wird;
- oder ein Gemisch ist, das auf 100 Mol-% aus Folgendem besteht:
- Wasser in einem Anteil von über 0 Mol-% und unter oder gleich 15 Mol-%, vorzugsweise unter oder gleich 5 Mol-%, und
- einem Keton, das aus Propan-2-on, Butan-2-on, Pentan-2-on, 3-Methyl-butan-2-on, 3-Ethyl-pentan-2-on oder 4-Methyl-pentan-2-on mit der Formel (II), wie zuvor definiert, ausgewählt wird, in einem molaren Anteil von über oder gleich 90 Mol-%, vorzugsweise über oder gleich 95 Mol-% und unter 100 Mol-%.

5. Verfahren nach Anspruch 4, wobei das Lösungsmittel (S) entweder Aceton oder ein Wasser-Aceton-Gemisch mit einem Molverhältnis Wasser/Aceton von über 0 und unter oder gleich 5/95 ist.

6. Verfahren nach einem der Ansprüche 1 bis 5, **dadurch gekennzeichnet, dass** der anionische Polyelektrolyt auf 100 Mol-% zwischen 0,05 Mol-% und 5 Mol-% und insbesondere zwischen 0,1 Mol-% und 1 Mol-% monomere Einheiten aufweist, die von einer Verbindung mit der Formel (I) stammen.

7. Verfahren nach einem der Ansprüche 1 bis 6, **dadurch gekennzeichnet, dass** der anionische Polyelektrolyt monomere Einheiten aufweist, die von einem neutralen Monomer stammen, das aus Acrylamid, 2-Hydroxyethylacrylat oder N,N-Dimethylacrylamid ausgewählt wird.

8. Verfahren nach einem der Ansprüche 1 bis 7, **dadurch gekennzeichnet, dass** der anionische Polyelektrolyt auf 100 Mol-% mehr als 10 Mol-%, vorzugsweise mehr als 25 Mol-% und bis zu 90 Mol-% monomere Einheiten, die von 2-Methyl-2-[(1-oxo-2-propenyl)amino]-1-propansulfonsäure stammen, die in Form des Natriumsalzes vollständig zu Salz umgewandelt ist, von 5 Mol-% bis zu weniger als 90 Mol-%, vorzugsweise bis zu weniger als 75 Mol-% eines neutralen Monomers und von 0 Mol-% bis 5 Mol-% eines Monomers mit der Formel (I) aufweist.

9. Verfahren nach Anspruch 8, **dadurch gekennzeichnet, dass** der anionische Polyelektrolyt auf 100 Mol-% mehr als 50 Mol-%, vorzugsweise mehr als 75 Mol-% und bis zu 85 Mol-% monomere Einheiten, die von 2-Methyl-2-[(1-oxo-2-propenyl)amino]-1-propansulfonsäure stammen, die in Form des Natriumsalzes vollständig zu Salz umgewandelt ist, von 10 Mol-% und bis zu 50 Mol-%, vorzugsweise weniger als 25 Mol-% eines neutralen Monomers und von 0 Mol-% bis 5 Mol-% eines Monomers mit der Formel (I) aufweist.

10. Verfahren nach einem der Ansprüche 1 bis 9, **dadurch gekennzeichnet, dass** der anionische Polyelektrolyt mit einer Diethylen- oder Polyethylenverbindung im molaren Anteil, ausgedrückt im Verhältnis zur Gesamtheit der eingesetzten Monomere, zwischen 0,005 Mol-% und 1 Mol-% vernetzt und/oder verzweigt wird.

11. Variante des Verfahrens nach einem der Ansprüche 1 bis 10, **dadurch gekennzeichnet, dass** sie statt Schritt a) einen Schritt a2 zum Herstellen eines Reaktionsgemischs, das in den gewünschten Verhältnissen und in einem Lösungsmittel (S) das oder die neutrale(n) Monomer(e), das Natriumsalz der 2-Methyl-2-[(1-oxo-2-propenyl)amino]-1-propansulfonsäure, gegebenenfalls das oder die Monomere mit der Formel (I), wie zuvor definiert, umfasst.

12. Variante des Verfahrens nach Anspruch 11, **dadurch gekennzeichnet, dass** sie vor Schritt a2) einen Schritt a1) zum Herstellen des Natriumsalzes der 2-Methyl-2-[(1-oxo-2-propenyl)amino]-1-propansulfonsäure durch Neutralisation der 2-Methyl-2-[(1-oxo-2-propenyl)amino]-1-propansulfonsäure mit einem Neutralisationsmittel umfasst, das in der Lage ist, das Natriumsalz der 2-Methyl-2-[(1-oxo-2-propenyl)amino]-1-propansulfonsäure zu bilden.

13. Verfahren oder seine Variante nach einem der Ansprüche 1 bis 12, **dadurch gekennzeichnet, dass** der vernetzte anionische Polyelektrolyt ein vernetztes Terpolymer aus der 2-Methyl-2-[(1-oxo-2-propenyl)amino]-1-propansulfonsäure, die in Form des Natriumsalzes vollständig zu Salz umgewandelt ist, aus N,N-Dimethylacrylamid und dem Methacrylat von pentacosaethoxyliertem Behenylalkohol ist, wobei auf 100 % monomere Einheiten mehr als 75 Mol-% und bis zu 85 Mol-% monomere Einheiten von 2-Methyl-2-[(1-oxo-2-propenyl)amino]-1-propansulfonsäure stammen, die in Form des Natriumsalzes vollständig zu Salz umgewandelt ist, von 10 Mol-% bis zu weniger als 25 Mol-% aus einem neutralen Monomer und 0,05 Mol-% und 5 Mol-% aus einem Monomer mit der Formel (I).
